(19) Europäisches Patentamt
European Patent Office
Office européen des brevets

(11) EP 1 840 225 A1

(12) EUROPEAN PATENT APPLICATION

(43) Date of publication:
**03.10.2007 Bulletin 2007/40**

(51) Int Cl.:
***C12Q 1/68*** (2006.01)

(21) Application number: **06255535.4**

(22) Date of filing: **27.10.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **30.03.2006 JP 2006095599**

(71) Applicant: **KABUSHIKI KAISHA TOSHIBA**
**Tokyo 105-8001 (JP)**

(72) Inventors:
- **Takahashi, Masayoshi**
  **c/o Toshiba Corporation**
  **Tokyo 105-8001 (JP)**
- **Gemma, Nobuhiro**
  **c/o Toshiba Corporation**
  **Tokyo 105-8001 (JP)**

(74) Representative: **Robertson, James Alexander**
**Marks & Clerk**
**90 Long Acre**
**London WC2E 9RA (GB)**

Remarks:
The sequence listing, which is published as annex to the application documents, was filed after the date of filing. The applicant has declared that it does not include matter which goes beyond the content of the application as filed.

(54) **Method of measuring the repeat number in a nucleic acid target**

(57) There is provided a method of measuring a repeat number of a unit base, the method comprising, when a repeat number can be possessed by a target nucleic acid is A or B (where A < B), and (B-A) /B < 0.20, using at least one first nucleic acid probe having a repeat number selected from a value of A-p, as well as at least one second nucleic acid probe having a repeat number selected from a value of B+s [where p and s are natural numbers satisfying 0.20 ≤ {(B+s)-(A-p)} /(B+s) ≤ 0.50]. The probes may be immobilised on an electrode array.

FIG. 5A

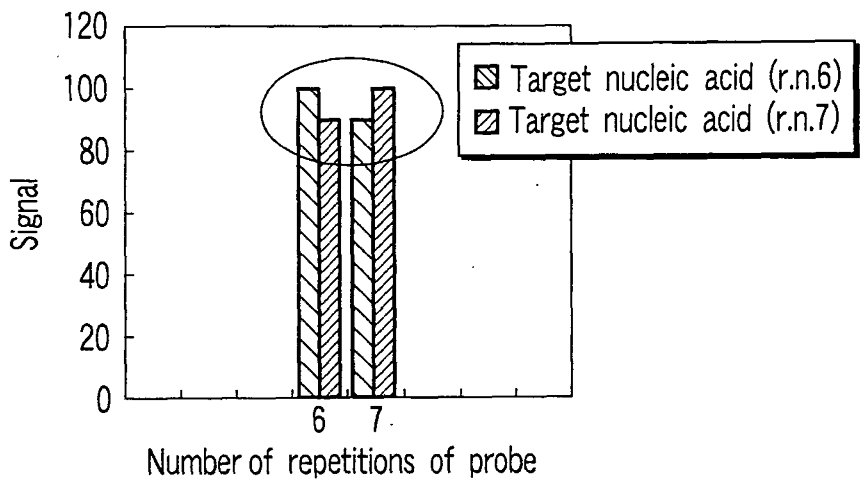


EP 1 840 225 A1

FIG. 5B

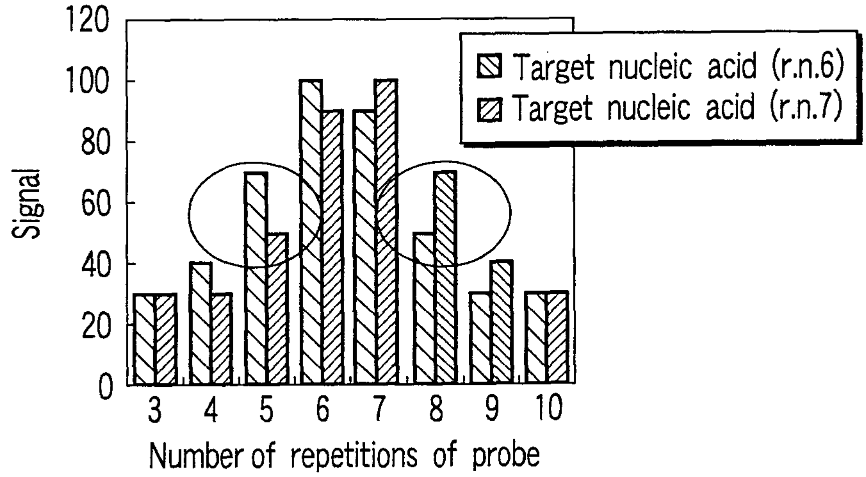
Signal
120
100
80
60
40
20
0
3 4 5 6 7 8 9 10
Number of repetitions of probe
Target nucleic acid (r.n.6)
Target nucleic acid (r.n.7)

## Description

The present invention relates to a method of measuring a repeat number of a unit base possessed by a repetitive region in a nucleotide sequence.

In recent years, analysis of an individual gene sequence to determine gene polymorphism has been applied in a variety of fields. For example, it has become possible to predict reactivity of a person to a drug in the medical field, and perform identification of an individual and make a comparison between an evidence sample and a suspected person in the forensic field. There are two kinds of gene polymorphisms which are said to be useful currently, and one of them is one base polymorphism in which a base at one place in a DNA sequence is replaced with another base. The other is a repeat number of a unit base in a "repeated sequence" in which a unit base consisting of two to several tens of bases is repeated a few to several tens of times, and this is called satellite polymorphism. Since a repeat number is different for every individual, it can be advantageously used in a variety of analyses (see, for example, JP-A 1999-503019(KOKAI)).

As for satellite polymorphism, for example, in the medical field, side effect of irinotecan administration has been predicted by detecting a repeat number of a unit base which is repeated in the UGT1A1 gene. In addition, in the forensic field, a procedure has been standardized which identifies an individual and verifying with an evidence sample by determining a repeat number of microsatellite polymorphism of 15 regions.

For measuring such polymorphism, a microarray such as a DNA chip is preferably used. Conventionally, in a DNA chip for identifying a repeat number, a nucleic acid probe having the same repeat number as that of a target nucleic acid to be detected has been used.

However, even when a repeat number of target nucleic acid is different from a repeat number of a nucleic acid probe, an excessive base is, in some cases, looped out, resulting in binding. In some cases, a binding force in this case is the same extent of that of a binding force when repeat numbers of a target nucleic acid and a nucleic acid probe are the same. For this reason, there has been a problem that determination of a repeat number is difficult.

An object of the present invention is to provide a method of measuring a repeat number of a unit base possessed by a repetitive region in a target nucleotide sequence, simply and at a high precision.

According to one aspect of the present invention, there is provided a method of measuring a repeat number of a unit base possessed by a repetitive region in a target nucleic acid sequence, the method comprising: a step of preparing a nucleic acid probe; a step of immobilizing the prepared nucleic acid probe on a substrate; a step of hybridizing the target nucleic acid to the nucleic acid probe immobilized on the substrate; a step of detecting the target nucleic acid which has been bound to the nucleic acid probe; and a step of determining a repeat number possessed by the target nucleic acid from results obtained in the detection step, wherein, the nucleic acid probe comprise a sequence complementary with a sequence of a repetitive region and its adjacent regions in the target nucleic acid sequence, and when a repeat number can be possessed by the target nucleic acid is A or B (where $A < B$), and $(B\text{-}A)/B < 0.20$, the nucleic acid probe contain at least one first nucleic acid probe having a repeat number selected from a value of A-p, as well as at least one second nucleic acid probe having a repeat number selected from a value of B+s [where p and s are natural numbers satisfying $0.20 \leq \{(B+s)-(A-p)\}/(B+s) \leq 0.50$].

The invention can be more fully understood from the following detailed description when taken in conjunction with the accompanying drawings, in which:

FIG. 1 is an outline view of a conventional measuring method;
FIG. 2 is a schematic view of results by the conventional measuring method;
FIG. 3 is an outline view of a measuring method according to one embodiment of the present invention;
FIG. 4 is a schematic view of results of the measuring method in one embodiment of the present invention;
FIGS. 5A and 5B are schematic views of measured values by each method; and
FIG. 6 is a view showing results of Examples of the present invention.

The present invention provides a method of measuring a repeat number of a unit base possessed by a repetitive region in a target nucleic acid sequence. In the present invention, the term "repetitive region" means a sequence part in which a unit base is repeated, present in a genome DNA sequence. Herein, the term "unit base" means a sequence consisting of a few to several tens of bases, each of which is to be a unit of repetition. For example, a unit base in minisatellite polymorphism includes over ten bases to several tens of bases, and a unit base in microsatellite polymorphism includes 2 to 4 bases. However, the number of bases included in a unit base is not limited thereto.

The term "repeat number" measured by the invention means the number of unit bases contained in the repetition region, that is, the number of repetitions of a unit base. Since a repeat number is different depending on an individual, a measured repeat number can be effectively utilized in the medical field or the forensic field.

Many repetitive regions are contained in a genome DNA, but a region for which a repeat number is measured may be arbitrarily determined. In the current DNA analysis, a particular region (STR: one kind of microsatellite; a unit base includes 4 bases) is mainly utilized, but the region may be determined according to various objects such as individual identification or investigation of disease association.

In the present invention, the term "target nucleic acid" means a nucleic acid having a repetitive region, for which measurement of a repeat number is desired. The target nucleic acid has a sequence of a repetitive region and its adjacent region (i.e., upstream and downstream regions), and the sequence is also referred to as target sequence.

The target nucleic acid is used as a sample solution containing target nucleic acid. A nucleic acid collected from a specimen may be used as it is, or a target sequence part may be amplified in advance by a procedure such as PCR, LAMP, or ICAN, which may be used as a target nucleic acid. Thereupon, a length of the target nucleic acid may be arbitrarily determined, and for example, may be a length of around 30 to 500 bps. A length of the target nucleic acid can be regulated by appropriately designing primers.

The target nucleic acid may have any structure of a straight structure and a loop structure. By suitably selecting a length and a structure of the target nucleic acid, efficiency of hybridization with a nucleic acid probe can be increased.

In order to detect a target sequence, a nucleic acid probe having a sequence complementary with that of the target nucleic acid is used in the measuring method of the invention. The nucleic acid probe is prepared according to the following conditions, and is used by being immobilized on a substrate. A sample solution which may contain a target nucleic acid is applied to a substrate on which a nucleic acid probe is immobilized like this, and hybridization between a nucleic acid probe and target nucleic acid is performed. A nucleic acid which has been bound to a nucleic acid probe in this reaction is a target nucleic acid having a desired sequence. By detecting to which nucleic acid probe the nucleic acid has bound, a repeat number possessed by the target nucleic acid can be determined.

In one embodiment, it is preferable that a nucleic acid probe is immobilized on an electrode placed on a substrate. Using an electrode makes it possible to electrochemically detect target nucleic acid bound to a nucleic acid probe. Single electrode is immobilized one kind of nucleic acid probes, and a plurality of electrodes may be placed on a same substrate.

As a length of a nucleic acid probe, a suitable length for immobilizing on a substrate and performing hybridization may be appropriately selected, and may be shorter than target nucleic acid. For example, the length may be about 3 to about 1000 bps, preferably about 10 to about 200 bps.

The nucleic acid probe for use in the invention is prepared so as to have a sequence complementary with a sequence of a repetitive region and its adjacent regions in the target nucleotide sequence. Herein, the term "complementary" means complementary in a range of 50% to 100%, provided that a sequence in a repetitive region is subject to the following conditions.

The present invention has an advantageous effect when $(B-A)/B < 0.20$ assuming that a repeat number can be possessed by target nucleic acid is A or B (where, $A < B$). A nucleic acid probe includes a sequence complementary with a sequence of a repetitive region and its adjacent regions in target nucleic acid as described above. However, a part of a repetitive region is not a completely complementary sequence. The probe contain at least one first nucleic acid probe having a repeat number selected from a value of A-p, as well as at least one second nucleic acid probe having a repeat number selected from a value of B+s [where, p and s are natural numbers satisfying $0.20 \leq \{(B+s)-(A-p)\}/(B+s) \leq 0.50$]. The first nucleic acid probe and the second nucleic acid probe each may be one kind, or may be two or more kinds.

Herein, an example of the case where a repeat number possessed by target nucleic acid is 6 or 7 will be explained with reference to the drawings. FIG. 1 is a view showing an outline of a conventional measuring method. In conventional methods, a nucleic acid probe having the same repeat number as that of the target nucleic acid, has been used. More specifically, hybridization with target nucleic acid 4 or 5 has been performed by using a nucleic acid probe 2 having a repeat number of 6 and a nucleic acid probe 3 having a repeat number of 7, which are immobilized on a solid phase electrode placed on a substrate 1.

However, as shown in FIG. 2, even when repeat numbers of target nucleic acid and a nucleic acid probe are different from each other, an excessive base may looped out and result in binding. Thereupon, a force of binding nucleic acids having different repeat numbers is reduced as compared with a force of binding nucleic acids in which repeat numbers of target and probe are the same. However, an extent of the reduction is slight, and therefore, the reduction of signal to be detected is also slight. A schematic view of a signal which is detected thereupon is shown in FIG. 5A.

FIG. 5A is a view showing the case where target nucleic acids respectively having a repeat number (r.n.) of 6 and 7 were detected with nucleic acid probes respectively having a repeat number of 6 and 7. The target nucleic acids having a repeat number of 6 are bound to the nucleic acid probes having a repeat number of 6 in many cases. While, they are also bound to the nucleic acid probe having a repeat number of 7 in many cases, so the difference between them is slight. Similarly, the target nucleic acids having a repeat number of 7 are bound to the nucleic acid probes having a repeat number of 7 in many cases, while are also bound to the nucleic acid probes having a repeat number of 6 in many cases, so the difference between them is slight. Therefore, according to the conventional method, it is difficult to determine a repeat number possessed by target nucleic acid.

On the other hand, according to the present method, at least one first nucleic acid probe having a repeat number selected from a value of A-p, and at least one second nucleic acid probe having a repeat number selected from a value of B+s [where p and s are natural numbers satisfying $0.20 \leq \{(B+s)-\{(A-p)/(B+s)\} \leq 0.50$] are used. Herein, A and B (where, A < B) are repeat numbers possessed by the target nucleic acid, and in this example, 6 and 7. In addition, p = 1, and s = 1.

From the above conditions, as shown in FIG. 3, the first nucleic acid probe is a nucleic acid probe 9 having a repeat number of 5, and a second nucleic acid probe is a nucleic acid probe 8 having a repeat number of 8. For easy understanding, a view of nucleic acid probes 2 and 3 respectively having repeat numbers of 6 and 7 is also described in FIG. 3.

FIG. 4 is a schematic view of the case where target nucleic acid is hybridized by using the above four kinds of nucleic acid probes. Like FIG. 2, using, as a standard, a binding force in the case of nucleic acids having the same repeat number, a binding force is remarkably decreased when a nucleic acid probe having a repeat number of 8 is bound to target nucleic acid having a repeat number of 6. In addition, also when a nucleic acid probe having a repeat number of 5 is bound to target nucleic acid having a repeat number of 7, a binding force is remarkably reduced likely. A schematic view of a signal detected thereupon is shown in FIG. 5B.

FIG. 5B is a view showing the case where target nucleic acids having repeat numbers (r.n.) of 6 and 7 are detected with a nucleic acid probe having a repeat number of 3 to 10. Herein, with regard to the target nucleic acid having a repeat number of 6, the target nucleic acid is bound to a nucleic acid probe having a repeat number of 5 more as compared with a nucleic acid probe having a repeat number of 8. The difference between them is more remarkable as compared with that between the nucleic acid probes having repeat numbers of 6 and 7.

On the other hand, with regard to the target nucleic acid having a repeat number of 7, a larger amount of the target is bound to a nucleic acid probe having a repeat number of 8 as compared with a nucleic acid probe having a repeat number of 5. The difference between them is more remarkable as compared with that between the nucleic acid probes having repeat numbers of 6 and 7.

Therefore, as explained herein, by using a nucleic acid probe having a different repeat number from a repeat number of target nucleic acid, a difference in a binding amount of target nucleic acid due to a difference in a nucleic acid probe becomes remarkable. Accordingly, a difference in signal due to difference in a nucleic acid probe becomes remarkable, and it is possible to more clearly decide a repeat number.

Like this, in the present invention, on the basis of a repeat number possessed by target nucleic acid, a difference in binding force due to a difference in repeat number can be made clear by using a nucleic acid probe satisfying the above conditions, so that a precision of measurement can be improved.

When a repeat number is measured according to the method of the invention, the ratio of absolute values of repeat numbers (herein A and B) can be possessed by target nucleic acid is important. For example, when $A = 6$ and $B = 7$, $(B-A)/B = 0.11$, so that discrimination is difficult as examples of FIGS. 1 and 2. On the other hand, $p = 1$ and $s = 1$ according to the present invention, $\{(B+S)-(A-p)\}/(B+S)= 0.30$, and discrimination becomes more clear as examples of FIGS. 3 and 4. Thereby, the present invention can obtain the advantageous effect when $(B-A)/B < 0.20$. By using at least one first nucleic acid probe having a repeat number selected from a value of A-B, and at least one second nucleic acid probe having a repeat number selected from a value of B+s, and by selecting p and s as being natural numbers satisfying $0.20 \leq \{(B+s)-(A-p)\}/(B+s) \leq 0.50$, a repeat number can be measured with better precision.

When $(B-A)/B$ is small, specifically, when it is less than 0.20, discrimination is difficult according to the conventional method. However, by applying the equation $\{(B+s)-(A-p)\}/(B+s)$ of the present invention, this value can be made to be 0.2 or more. That is, a ratio of a difference between two repeat numbers to be discriminated (i.e., B-A) relative to a greater repeat number (i.e., (B-A)/B) can be made to be greater by applying the equation $\{(B+s)-(A-p)\}/(B+s)$ of the present invention.

Consequently, by using probe nucleic acids having repeat numbers of A-p and B+s, reduction in a binding force can be more remarkable as compared with the case where probe nucleic acid and target nucleic acids have the same repeat number. For this reason, by detecting an amount of target nucleic acid bound to the probe, it is possible to more clearly discriminate a repeat number of a nucleic acid. Note that, when a value of $\{(B+s)-(A-p)\}/(B+s)$ is too large, that is, when a different between B and A is large, the effect of the invention is reduced. Therefore, it is preferable that $\{(B+s)-(A-p)\}(B+s) \leq 0.5$.

In the present invention, detection of target nucleic acid hybridized with a nucleic acid probe may be performed by an arbitrary method; preferably it may be detected by electrochemical method. An amount of target nucleic acid bound to both kind of nucleic acid probe can be detected by, for example, a current value. From results of detection of both, a repeat number of target nucleic acid is determined. Specifically, results obtained from the first nucleic acid probe are compared with results obtained from the second nucleic acid probe, and a value of A or B applied to a probe to which more target nucleic acids are bound is a repeat number possessed by target nucleic acid.

Further, when target nucleic acid may have three or more kinds of different repeat numbers, a combination of arbitrary two kinds of them is made, and in all the combinations, a nucleic acid probe is prepared so as to satisfy the conditions of the present invention.

A substrate which can be used in the invention may be any substrate on which a nucleic acid probe can be immobilized. The substrate may be a plate-like form having for example, a well, a groove or a flat surface or a steric shapes such as a sphere, made of for example, a non-porous, hard or semi-hard material. A substrate can be prepared from a silica-containing substrate such as silicon, glass, quartz glass or quartz, or plastic or a polymer such as polyacrylamide, polystyrene, or polycarbonate, without any limitation.

For the nucleic acid probe-immobilized substrate of the present invention, an electrochemically method can be, without limitation, applied as means for sensing the presence of a duplex produced from hybridization between a nucleic acid probe immobilized on the substrate and target nucleic acid.

When double-stranded nucleic acid is detected by an electrochemically method, an electrode is placed on a substrate, and a nucleic acid probe is immobilized on the electrode. Although not limited thereto, the electrode can be formed of a carbon electrode such as graphite, glassy carbon, pyrolytic graphite, carbon paste, or carbon fiber; a noble metal electrode such as platinum, platinum black, gold, palladium, or rhodium; an oxide electrode such as titanium oxide, tin oxide, manganese oxide, or lead oxide; a semiconductor electrode such as Si, Ge, ZnO, CdS, $TiO_2$, or GaAs; titanium or the like. These electrodes may be coated with a conducting polymer, may be coated with a single molecular membrane, or may be treated with other surface treating agent, if desired.

Detection of double-stranded nucleic acid by an electrochemical method may be performed by using, for example, a known double-stranded nucleic acid recognizing substance. Herein, the double-stranded nucleic acid recognizing substance means a molecule which has electrochemical activity and which is bound to nucleic acid. Although not limited thereto, examples of the duplex recognizing substance include Hoechst 33258, Acridine Orange, quinacrine, daunomycin, a metallointercalator, a bisintercalator such as bisacridine, a trisintercalator or a polyintercalator. Further, it is also possible to modify these intercalators with an electrochemically active metal complex, for example, ferrocene or viologen in advance. Alternatively, other known double-stranded nucleic acid recognizing substances may be used.

Immobilization of a nucleic acid probe may be performed by known means. For example, by fixing a spacer on an electrode, and immobilizing a nucleic acid probe on the spacer, the nucleic acid probe may be immobilized on the electrode via the spacer. Further, a spacer is bound to a nucleic acid probe in advance, and the probe may be immobilized on an electrode via the spacer. Alternatively, a spacer and a nucleic acid probe may be synthesized on an electrode by known means. In addition, for immobilizing a nucleic acid probe via a spacer, the spacer may be directly immobilized on a treated or non-treated electrode surface by covalent bond, ion bond or physical adsorption. Alternatively, a linker agent which aids immobilization of a nucleic acid probe via a spacer may be used. An electrode may be treated with a blocking agent for preventing non-specific binding of nucleic acid to an electrode together with a linker agent. Moreover, a linker agent and a blocking agent used herein may be a substance for advantageously performing electrochemically detection.

Further, like other general electrochemical methods, a substrate may be provided with a counter electrode and/or a reference electrode. When a reference electrode is arranged, a general reference electrode such as a silver/silver chloride electrode and a mercury/mercury chloride electrode can be used.

Measurement of a repeat number according to the present invention can be performed, in one embodiment, as follows.

First, a nucleic acid component is extracted as sample nucleic acid from a sample collected from a subject such as an individual such as an animal including a human, a tissue or a cell. The resulting sample nucleic acid may be subjected to treatment such as reverse transcription, extension, amplification and/or enzyme treatment, if necessary. Sample nucleic acid which has been pre-treated if necessary is contacted with a nucleic acid probe immobilized on a substrate, to react them under the conditions permitting suitable hybridization. Such suitable conditions can be appropriately selected by a person skilled in the art, depending on various conditions such as the kind of base contained in a target sequence, kinds of spacer and nucleic acid probe immobilized on the substrate, kind of sample nucleic acid and the states thereof.

A hybridization reaction may be performed, for example, under the following conditions.

A hybridization reaction solution uses a buffer having an ionic strength in a range of 0.01 to 5, and a pH in a range of 5 to 10. To this solution, dextran sulfate which is a hybridization promoter, as well as a salmon sperm DNA, a bovine thymus DNA, EDTA, and a surfactant etc. may be added. The resulting sample nucleic acid is added thereto, and this is thermally denatured at 90°C or higher. A nucleic acid probe-immobilized substrate is inserted into the solution immediately after denaturation of nucleic acid, or after rapid cooling to 0°C. Alternatively, a hybridization reaction may be performed by adding a solution dropwise to the substrate.

During a reaction, a reaction rate may be enhanced by a procedure such as stirring and shaking. A reaction temperature may be, for example, in a range of 10 to 90°C, and a reaction time may be 1 minute or longer, overnight. After a hybridization reaction, an electrode is washed. As a cleaning solution, for example, a buffer having a pH in a range of 5 to 10, and an ionic strength in a range of 0.01 to 5 is used. When target nucleic acid containing a target sequence is present in sample nucleic acid, this is hybridized with a nucleic acid probe to produce a double-stranded nucleic acid on a substrate.

Subsequently, double-stranded nucleic acid is detected by electrochemical means. As a detecting procedure, generally, a substrate is washed after a hybridization reaction, and a duplex recognizing substance is made act on a double-stranded part formed on an electrode surface to measure a signal generated therefrom electrochemically.

The concentration of the duplex recognizing substance is different depending on its kind, and the substance is generally used in a range of 1 ng/mL to 1 mg/mL. Thereupon, a buffer having a pH in a range of 5 to 10, and an ionic strength in a range of 0.001 to 5 may be used.

Electrochemical measurement is possible, for example, by applying a potential not lower than a potential at which a duplex recognizing substance is electrochemically reacted, and measuring a reaction current value derived from the duplex recognizing substance. Thereupon, a potential may be scanned at a constant rate, or may be applied as a pulse, or a constant potential may be applied. Upon measurement, for example, a device such as a potentiostat, a digital multimeter or a function generator may be used to control current or voltage. Further, the concentration of target nucleic acid may be calculated from a calibration line on the basis of the resulting current value. The resulting results are compared, and a nucleic acid probe to which more target nucleic acids are bound is determined. Thereby, a repeat number in target nucleic acid is determined.

Also, according to another aspect of the invention, a nucleic acid probe-immobilized substrate for use in a method of measuring a repeat number is provided. The nucleic acid probe-immobilized substrate in the invention is a substrate on which the aforementioned nucleic acid probe is immobilized. The substrate immobilized nucleic acid probe of the invention may have an arbitrary shape, but a board-like substrate may be preferably used, and is herein also referred to as array or chip.

The term "nucleic acid" used herein is a term comprehensively indicating nucleic acid and a nucleic acid analog, such as ribonucleic acid (RNA), deoxyribonucleic acid (DNA), peptide nucleic acid (PNA), methylphosphonate nucleic acid, S-oligo, cDNA and cRNA, as well as any oligonucleotide and polynucleotide. Such a nucleic acid may be naturally occurred, or may be artificially synthesized. Herein, a nucleotide sequence may be a genome DNA sequence, or may be a fragmental sequence by which a whole genome is not maintained.

The method of measuring a repeat number of a unit base according to the method of the invention can be used, for example, in drug efficacy determination (effect or side effect at drug administration), and individual identification (identification for confirming person himself, parentage test, etc.), but not limited thereto. Individual identification means determination consistency between a nucleotide sequence possessed by an individual and a nucleotide sequence of a sample. For example, the method can be used for specifying that an article left such as a remaining bloodstain and hair is of a suspected or a victim in criminal investigation. Therefore, a nucleotide sequence as a sample may be nucleic acid contained in those articles left, but not limited thereto. The subject is preferably a human, and additionally, may include livestock and pets, or cultivated or wild plants. Individual identification can be performed by comparing a repeat number determined by the method of the invention between samples, and determining whether both are consistent or not.

As described above, according to the present invention, a repeat number of a unit base possessed by a repetitive region in a target nucleic acid sequence can be measured simply and at high precision.

## Examples

An example of detection of target nucleic acid in which a unit base includes two bases (TA) and a repeat number is 8 or 9 will be described below.

<Target nucleic acid sequence>

A nucleic acid chain having the following sequence is used as target nucleic acid.

5′GGAATCAGCTGCCCAGATACAAAGATGGGATTCAGGTGGCAGATGGACCC[TA
]nGAAGAGGACATGGAGAGAAAGAGGAAGCTCCTACAGACAC3′

wherein, n = 8 (SEQ ID No: 1) or n = 9 (SEQ ID No: 2).

<Nucleic acid probe>

A nucleic acid probe having a sequence complementary with a sequence containing a repetitive region and its vicinity regions in target nucleic acid was set as follows:

5' CATGTCCTCTTC[TA]nGGGTCCATCTG 3'

wherein, n = 5, 6, 7, 8, 9, 10, 11, or 12 (SEQ ID Nos: 3 to 10, respectively). Herein, n = 8 and 9 are conventional probes, and n = 5 to 7 and 10 to 12 are nucleic acid probes used according to the present invention.

<Substrate (DNA chip)>

In the present Example, an electrode substrate as a DNA chip in which a plurality of gold electrodes were arranged on one glass substrate. In addition to an acting electrode to which a probe is immobilized, on the electrode substrate as a DNA chip, a reference electrode and a counter-electrode, as well as an electrode pad for detecting a current value which is wired to each electrode are placed.

<Preparation of nucleic acid probe-immobilized electrode>

A 3'-terminus of the nucleic acid probe sequence was modified with a thiol (SH) group, and a solution containing this was used as a nucleic acid probe solution. Various nucleic acid probe solutions were added dropwise to respective electrodes placed on the DNA chip, and these was allowed to stand to immobilize various nucleic acid probes to respective electrodes.

<Detection of target nucleic acid>

A solution containing target nucleic acid was added such that the nucleic acid probe electrode was covered, and this was allowed to stand to permit target nucleic acid to be bound (hybridized) to a nucleic acid probe. Thereafter, this was washed with a suitable buffer in order to remove non-specifically absorbed or bound target nucleic acids. After the buffer for washing was removed, Hoechst 33258 as an intercalator which binds to nucleic acid was added. At that time point, a potential was scanned on an acting electrode, and an intercalator was oxidized to detect a current value.

Addition of a target nucleic acid solution, allowing to stand at a set temperature, addition of a buffer for washing, allowing to stand at a set temperature, addition of an intercalator, current detection, and comparison of current values obtained from respective electrodes were performed by using a pumping control system, a temperature control system, an automated detecting device including a potentiostat, and a software for determining a repeat number from comparison between control and current values of respective units necessary for the above-described operations.

<Results>

Current values obtained from respective nucleic acid probe-immobilized electrodes are shown in FIG. 6. As the prior art, when probes (8, 9) having the same repeat number relative to target nucleic acid having a repeat number (r.n.) of 8 or 9 were used , a difference in current value depending on target nucleic acid was recognized, but the difference was slight.

On the other hand, when probes (7, 10) having a repeat number according to the present invention were used, a difference in current value depending on target nucleic acid was recognized, and the difference was greater as compared with use of the conventional probes (8, 9).

Like this, by using not a probe completely complementary with target nucleic acid, but a nucleic acid probe having a different repeat number from a repeat number in target nucleic acid, it is possible to more clearly measure a repeat number present in target nucleic acid.

## Claims

**1.** A method of measuring a repeat number of a unit base possessed by a repetitive region in a target nucleic acid, the method **characterized by** comprising:

a step of preparing a nucleic acid probe (8, 9);
a step of immobilizing the nucleic acid probe on a substrate (1);
a step of hybridizing the target nucleic acid (4 or 5) to the nucleic acid probe immobilized on the substrate;
a step of detecting the target nucleic acid which has been bound to the nucleic acid probe; and
a step of determining a repeat number possessed by the target nucleic acid from results obtained in the detection step,

wherein, the nucleic acid probe comprise a sequence complementary with a sequence of a repetitive region and its adjacent regions in the target nucleic acid sequence, and when a repeat number can be possessed by the target nucleic acid is A or B (where A < B), and (B-A)/B < 0.20, the nucleic acid probe contain at least one first nucleic acid probe having a at least one second nucleic acid probe having a repeat number selected from a value of B+s [where p and s are natural numbers satisfying

$$0.20 \leq \{(B+s)-(A-p)\}/(B+s) \leq 0.50].$$

2. The method according to claim 1, **characterized in that** the determining step comprise comparing an amount of target nucleic acid bound to the first nucleic acid probe and an amount of target nucleic acid bound to the second nucleic acid probe, and determining a repeat number as a result of the comparison.

3. The method according to claim 1, **characterized in that**, when the target nucleic acid may have three or more kinds of different repeat numbers, the condition as defined in claim 1 is satisfied for all of combinations consisting of two kinds of repeat numbers arbitrarily selected.

4. The method according to claim 1, **characterized in that** the nucleic acid probe is immobilized on an electrode placed on the substrate (1), and the target nucleic acid bound to the nucleic acid probe is electrochemically detected.

5. The method according to claim 4, **characterized in that** the electrochemical detection is performed by binding a molecule having the electrochemical activity and affinity for nucleic acid, to a double-stranded nucleic acid consisting of nucleic acid probe and target nucleic acid, and detecting a current value obtained by the oxidation-reduction reaction.

6. A nucleic acid probe-immobilized substrate for measuring a repeat number of a unit base possessed by a repetitive region in a target nucleotide, **characterized by** comprising:

   a substrate (1),
   the first nucleic acid probes immobilized on the substrate, and
   the second nucleic acid probes immobilized on the substrate,

   wherein the each of first and second nucleic acid probes have a sequence complementary with a sequence of a repetitive region and its adjacent regions in the target nucleic acid sequence, and when a repeat number can be possessed by the target nucleic acid is A or B (where $A < B$), and $(B-A)/B < 0.20$, the first nucleic acid probe have a repeat number selected from a value of A-p, and the second nucleic acid probe have a repeat number selected from a value of B+s [where, p and s are natural numbers satisfying

$$0.20 \leq \{(B+s)-(A-p)\}/(B+s) \leq 0.50].$$

7. The substrate according to claim 6, **characterized in that** the nucleic acid probe is immobilized on an electrode placed on the substrate.

8. The substrate according to claim 6, **characterized in that** the substrate is a DNA chip.

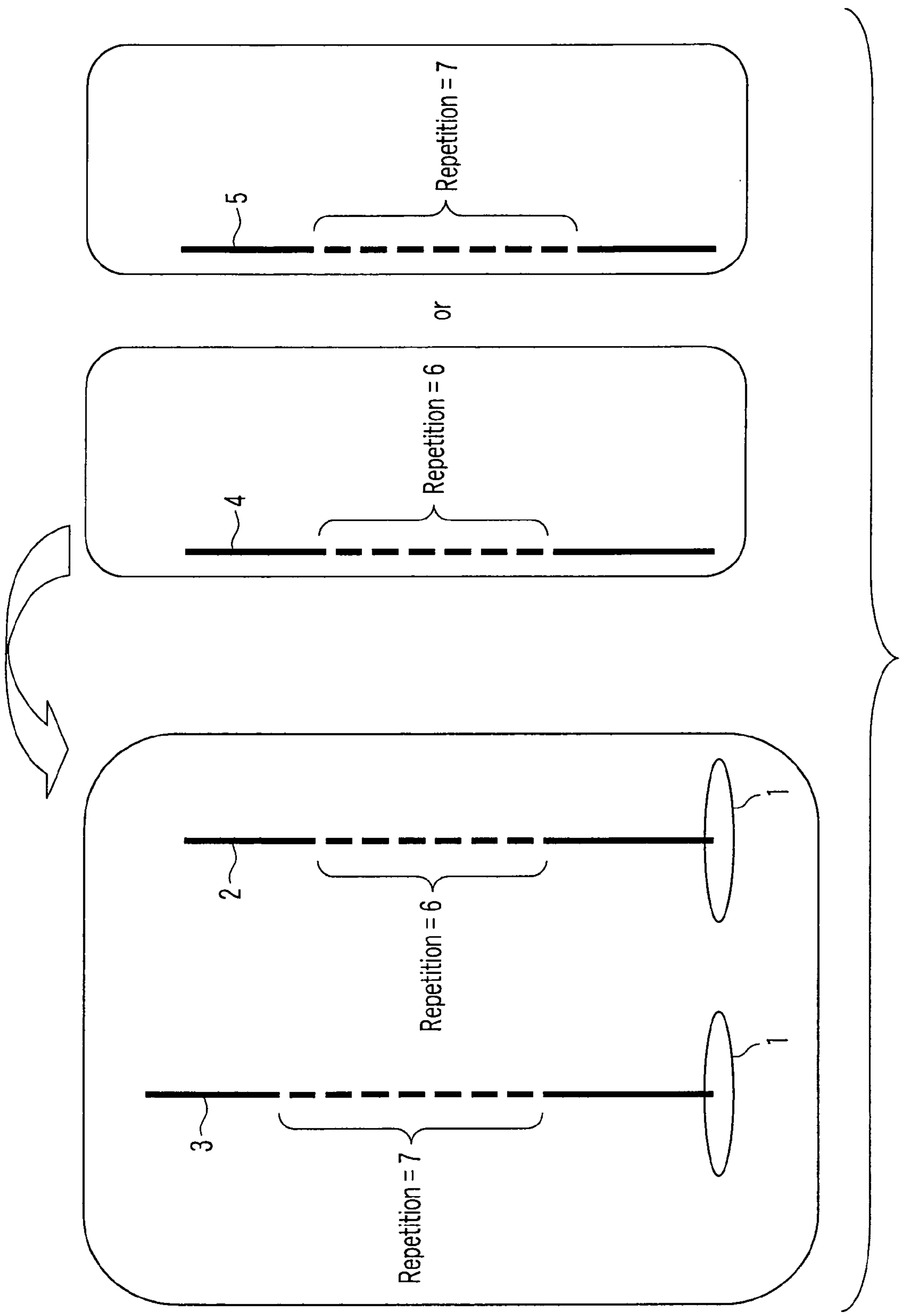
5
Repetition = 7
or
4
Repetition = 6
2
Repetition = 6
1
3
Repetition = 7
1

FIG. 1

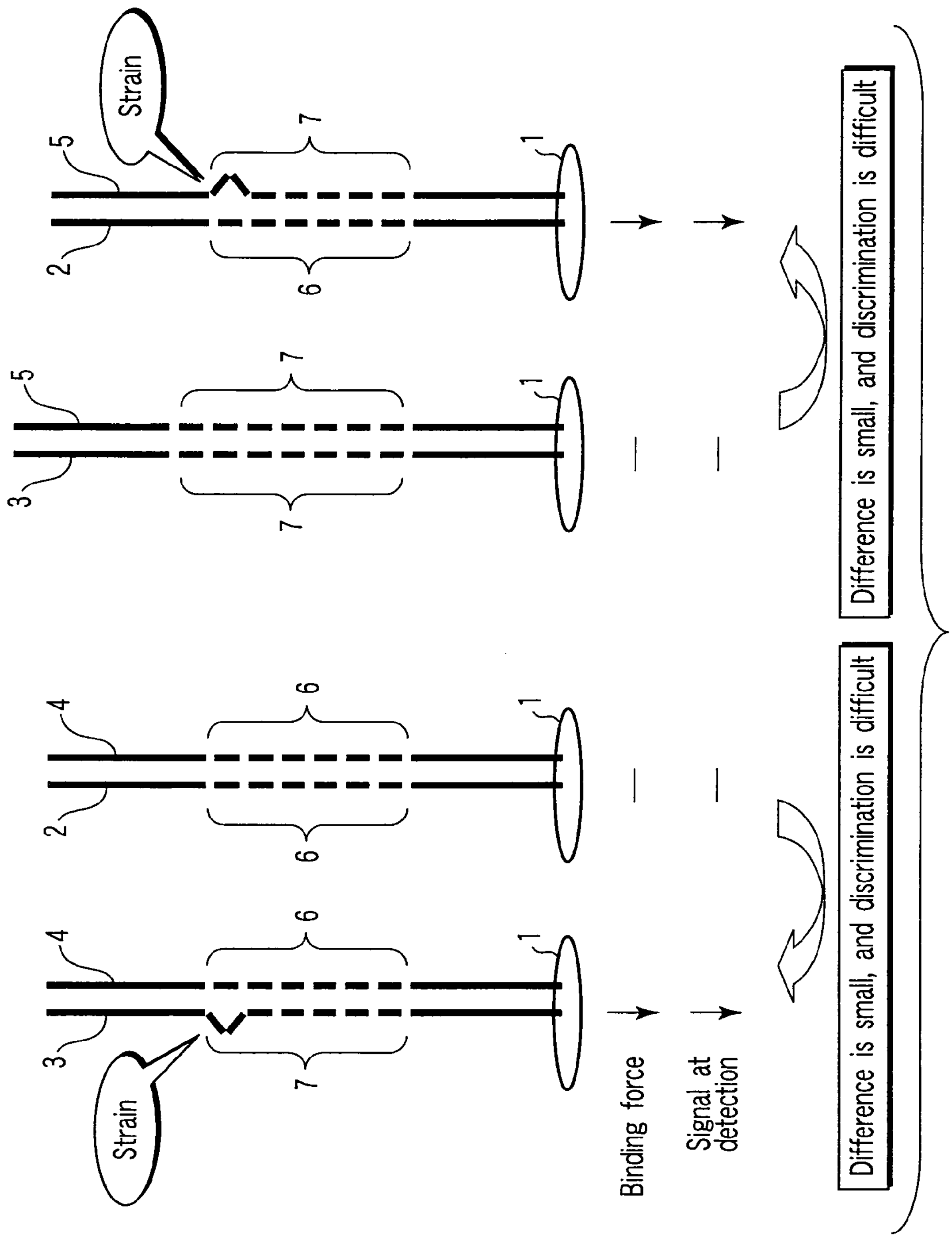
Strain
Strain
Binding force
Signal at detection
Difference is small, and discrimination is difficult
Difference is small, and discrimination is difficult
1
2
3
4
5
6
7

FIG. 2

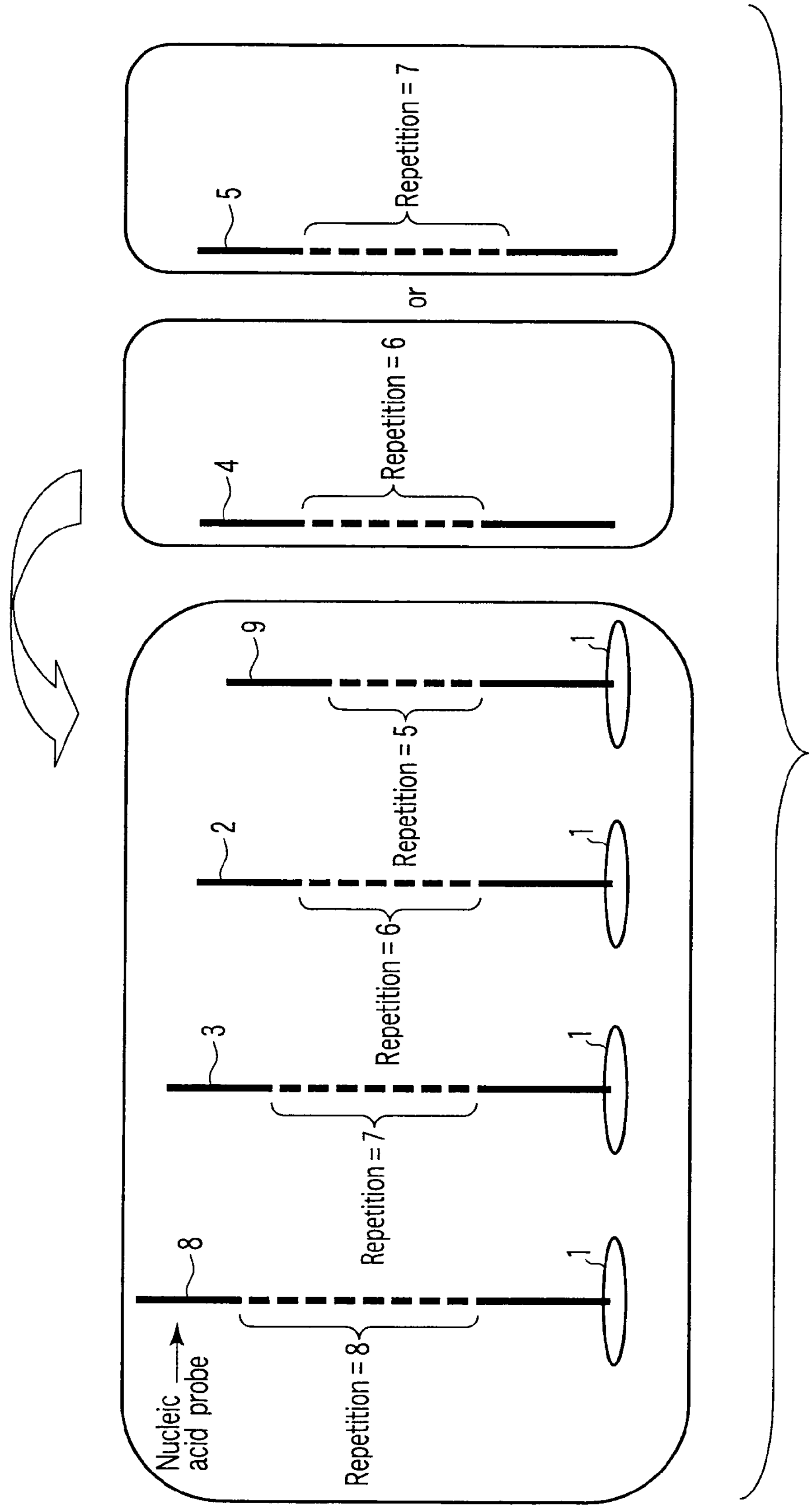
Nucleic acid probe
8
3
2
9
1
Repetition = 8
Repetition = 7
Repetition = 6
Repetition = 5
4
Repetition = 6
or
5
Repetition = 7

FIG. 3

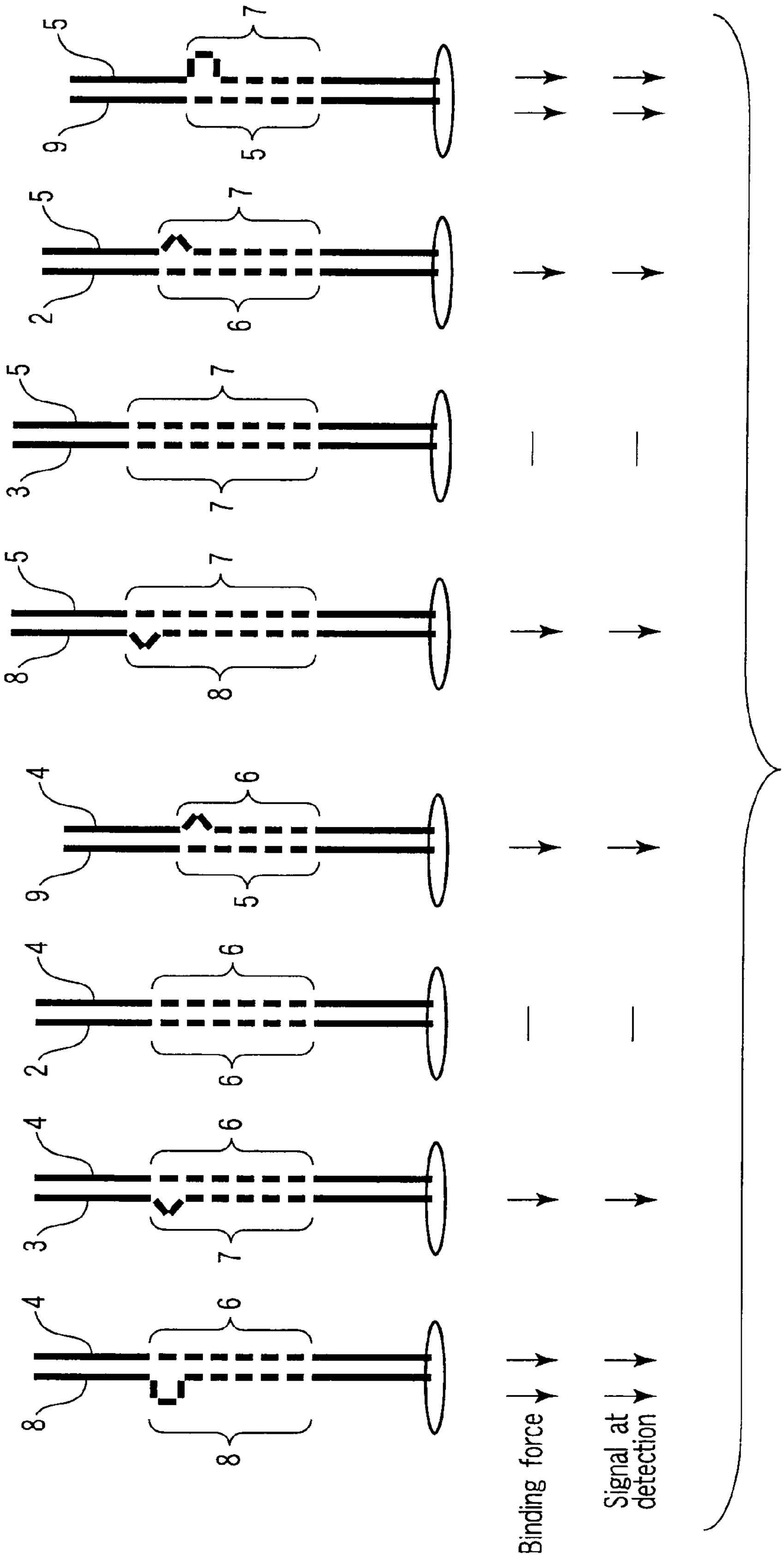


FIG. 4

FIG.5A

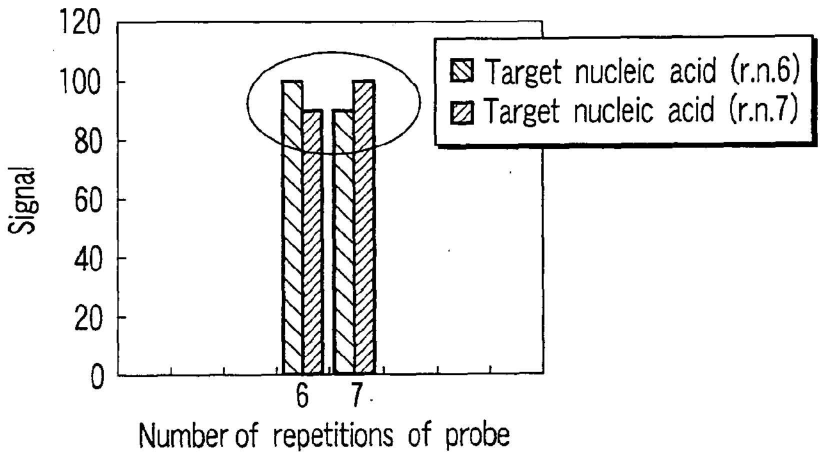


FIG.5B

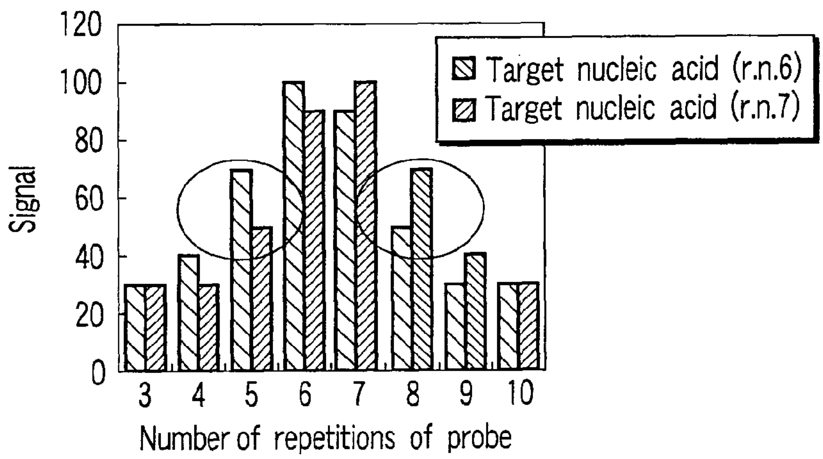


FIG.6

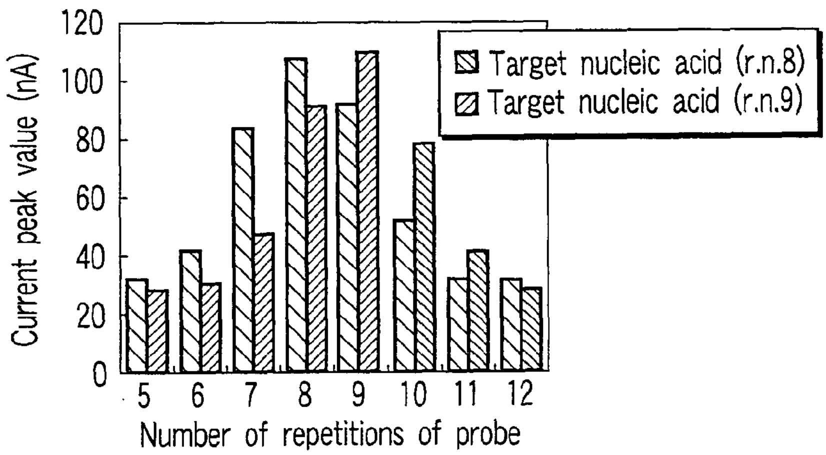

European Patent Office

# EUROPEAN SEARCH REPORT

Application Number
EP 06 25 5535

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 98/23776 A (AMERSHAM LIFE SCIENCE [GB]; REEVE MICHAEL ALAN [GB]) 4 June 1998 (1998-06-04)<br>* figure 1 *<br>----- | 1,3,6,8<br>2,4,5,7 | INV.<br>C12Q1/68 |
| X | WO 2005/113822 A (TRUSTEES OF THE LELAND BOARD O [US]; KEMP JENNIFER T [US]; WANG SHAN X) 1 December 2005 (2005-12-01)<br>* page 11; figure 1 *<br>----- | 1,3,6,8 | |
| X | WO 2005/014789 A (BIOARRAY SOLUTIONS LTD [US]) 17 February 2005 (2005-02-17)<br>* claim 15; figures 12A-C *<br>----- | 1 | |
| Y | WO 01/35100 A2 (CLINICAL MICRO SENSORS INC [US]) 17 May 2001 (2001-05-17)<br>* figures 4A-4C *<br>----- | 2,4,5,7 | |
| | | | TECHNICAL FIELDS SEARCHED (IPC) |
| | | | C12Q |

The present search report has been drawn up for all claims

3

| Place of search | Date of completion of the search | Examiner |
|---|---|---|
| Munich | 2 March 2007 | Knudsen, Henrik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 06 25 5535

This annex lists the patent family members relating to the patent documents cited in the above-mentioned European search report.
The members are as contained in the European Patent Office EDP file on
The European Patent Office is in no way liable for these particulars which are merely given for the purpose of information.

02-03-2007

| Patent document cited in search report | | Publication date | Patent family member(s) | | | Publication date |
|---|---|---|---|---|---|---|
| WO 9823776 | A | 04-06-1998 | US | 6083701 | A | 04-07-2000 |
| WO 2005113822 | A | 01-12-2005 | EP | 1753878 | A2 | 21-02-2007 |
| | | | US | 2006008823 | A1 | 12-01-2006 |
| WO 2005014789 | A | 17-02-2005 | NONE | | | |
| WO 0135100 | A2 | 17-05-2001 | AT | 269977 | T | 15-07-2004 |
| | | | AU | 778556 | B2 | 09-12-2004 |
| | | | AU | 1606301 | A | 06-06-2001 |
| | | | CA | 2388780 | A1 | 17-05-2001 |
| | | | DE | 60011821 | D1 | 29-07-2004 |
| | | | DE | 60011821 | T2 | 07-07-2005 |
| | | | EP | 1254372 | A2 | 06-11-2002 |
| | | | ES | 2225264 | T3 | 16-03-2005 |
| | | | JP | 3548159 | B2 | 28-07-2004 |
| | | | JP | 2003514227 | T | 15-04-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 1999503019 A

SEQUENCE LISTING

<110> Kabushiki Kaisha Toshiba

<120> METHOD OF MEASURING REPEAT NUMBER OF UNIT BASE

<130> EPP96152

<140> EP 06255535.4
<141> 2006-10-27

<150> JP 2006-095599
<151> 2006-03-30

<160> 10

<170> PatentIn version 3.3

<210> 1
<211> 106
<212> DNA
<213> Homo sapiens

<400> 1
ggaatcagct gcccagatac aaagatggga ttcaggtggc agatggaccc tatatatata 60

tatatagaag aggacatgga gagaaagagg aagctcctac agacac 106

<210> 2
<211> 108
<212> DNA
<213> Homo sapiens

<400> 2
ggaatcagct gcccagatac aaagatggga ttcaggtggc agatggaccc tatatatata 60

tatatataga agaggacatg gagagaaaga ggaagctcct acagacac 108

<210> 3
<211> 33
<212> DNA
<213> Artificial

<220>
<223> Probe

<400> 3
catgtcctct tctatatata tagggtccat ctg 33

<210> 4
<211> 35
<212> DNA
<213> Artificial

<220>
<223> Probe

<400> 4
catgtcctct tctatatata tatagggtcc atctg 35

<210> 5
<211> 37
<212> DNA
<213> Artificial

<220>
<223> Probe

<400> 5
catgtcctct tctatatata tatatagggt ccatctg 37

<210> 6
<211> 39
<212> DNA
<213> Artificial

<220>
<223> Probe

<400> 6
catgtcctct tctatatata tatatatagg gtccatctg 39

<210> 7
<211> 41
<212> DNA
<213> Artificial

<220>
<223> Probe

<400> 7
catgtcctct tctatatata tatatatata gggtccatct g 41

<210> 8
<211> 43
<212> DNA
<213> Artificial

<220>
<223> Probe

<400> 8
catgtcctct tctatatata tatatatata tagggtccat ctg 43

<210> 9
<211> 45
<212> DNA
<213> Artificial

<220>
<223> Probe

<400> 9
catgtcctct tctatatata tatatatata tatagggtcc atctg 45

<210> 10
<211> 47
<212> DNA
<213> Artificial

<220>
<223> Probe

<400> 10
catgtcctct tctatatata tatatatata tatatagggt ccatctg 47

SEQUENCE LISTING

<110> Kabushiki Kaisha Toshiba

<120> Methods for determination of the repetition frequency of a unit base

<130> EPP96152

<140> EP Unknown
<141>

<150> JP 2006-095599
<151> 2006-03-30

<160> 10

<170> PatentIn version 3.3

<210> 1
<211> 106
<212> DNA
<213> Human

<400> 1
ggaatcagct gcccagatac aaagatggga ttcaggtggc agatggaccc tatatatata 60

tatatagaag aggacatgga gagaaagagg aagctcctac agacac 106

<210> 2
<211> 108
<212> DNA
<213> Human

<400> 2
ggaatcagct gcccagatac aaagatggga ttcaggtggc agatggaccc tatatatata 60

tatatataga agaggacatg gagagaaaga ggaagctcct acagacac 108

<210> 3
<211> 33

<212> DNA
<213> Probe

<400> 3
catgtcctct tctatatata tagggtccat ctg 33

<210> 4
<211> 35
<212> DNA
<213> Probe

<400> 4
catgtcctct tctatatata tatagggtcc atctg 35

<210> 5
<211> 37
<212> DNA
<213> Probe

<400> 5
catgtcctct tctatatata tatatagggt ccatctg 37

<210> 6
<211> 39
<212> DNA
<213> Probe

<400> 6
catgtcctct tctatatata tatatatagg gtccatctg 39

<210> 7
<211> 41
<212> DNA
<213> Probe

<400> 7
catgtcctct tctatatata tatatatata gggtccatct g 41

<210> 8

<211> 43
<212> DNA
<213> Probe

<400> 8
catgtcctct tctatatata tatatatata tagggtccat ctg 43

<210> 9
<211> 45
<212> DNA
<213> Probe

<400> 9
catgtcctct tctatatata tatatatata tatagggtcc atctg 45

<210> 10
<211> 47
<212> DNA
<213> Probe

<400> 10
catgtcctct tctatatata tatatatata tatatagggt ccatctg 47